# EUROPEAN PATENT APPLICATION

(11) **EP 3 441 067 A1**
(43) Date of publication of application: **13.02.2019**
(21) Application number: 16897484.8
(22) Date of filing: 06.04.2016
(51) Int. Cl.: A61K 31/4375, A61K 31/122, A61P 35/00

(54) **ANTI-TUMOR COCKTAIL COMPRISING AN ANTI-TUMOR AGENT SELECTIVE FOR TUMOR ACIDITY, AN ANTIOXIDANT, AND A P-GLYCOPROTEIN INHIBITOR**

(71) Applicant: Arion Tecnologia Brasil - Gestão de Ativos S/A, 60150-161 Fortaleza - CE (BR)
(72) Inventor: SILVA PAIVA, Gerson, CEP-22410-003 Rio de Janeiro (BR)
(74) Representative: Durán-Corretjer, S.L.P.
(86) International application number: PCT/BR2016/000037
(87) International publication number: WO 2017/173509

(57) **Abstract**

The present invention relates to an anti-tumor cocktail comprising a combination of a commercial anti-tumor drug (1), such as 9-acridinecarboxylic acid, in association with an antioxidant (2), such as Coenzyme Q10, and a P-glycoprotein inhibitor (3), such as metformin, ketoconazole, quinidine, verapamil, cyclosporin, ketoconazole, erythromycin, spironolactone, itraconazole, tariquidar, zosuquidar, elacridar, laniquidar, ontogen, imidazole, anthranilamide, dexverapamil and/or dexniguldipine, with a view to selectively destroying solid tumor cells without affecting rapidly replicating healthy cells.

## Description

The present invention refers to a cocktail to combat all types of solid cancers, called here "Anti-tumor cocktail", which comprises an anti-tumor agent selective for tumor acidity, an antioxidant and a P-glycoprotein inhibitor. This cocktail is able to selectively destroy cells of solid tumors effectively without affecting healthy cells of rapid replication.

The first chemotherapy against cancer was acridine. Acridine (di-benzene [b,e] pyridine or 10-azaanthracene) was isolated by Graebe in 1871; isolated from high boiling point oils in 1926, by Wirth (Patent DE 440771); prepared from the N-phenylantranilic acid by Perkin, in 1923 (Patent GB 214756); from Ca-antranilate by Koller in 1928 (Monatsh. 50,51); passing the steam of benzylaniline by a hot platinum wire by Myer, in 1916 (Monatsh., 37,698) and by Ullmann, in 1907 (Ber. 40, 2521). Lhome et al. (Patent application WO 94/25439, of 14 November 1994 prepared substances derived from acridine with antineoplastic properties, such as the (3,6-bis-dimethylamino-acridin-4-il)-methanol. Ferlin et al. (Eur. J. Med. Chem. 35:827-837, 2000) prepared substituted derivatives of 9-acridine and azacridine (m-AMSA analogues) with anti-tumoral activity containing inhibitory effect on DNA-topoisomerase II. Although these acridins present selective activity for DNA-topoisomerase, they are known to exhibit high toxicity, destroying tumor and healthy cells at the same time. None of the documents cited above presented compounds with high selectivity to tumors. Currently, more active and less toxic chemotherapies are available for use in clinical practice. Chemotherapy works by interrupting or slowing down the growth of cancer cells, which grow and divide rapidly. However, chemotherapy can also damage healthy cells that divide rapidly, such as those in the lining of the mouth and intestine, the white blood cells, responsible for the defense, and the ones that make the hair grow, besides being able to often result in renal and cardiac complications. The main objective in the development of antineoplastic chemotherapeutic agents is the improvement of selectivity and the consequent reduction of undesirable side effects. To reduce its side effects due to non-selectivity of these antineoplastic drugs, these are chemically bonded to sugar molecules, since malignant tumors require about ten times more glucose than healthy cells. An example of antineoplastic drugs that are carried by glucose are D-19575, also known as glufosfamide, published in the US Patent No. 5 622 936, and derivatives of glucose-chlorambucila (Halmos, T., Santarromana, M., Antonakis, K., Sherman, D. European Journal of Pharmacology: 318:477-484, 1996). However, these medications end up affecting healthy cells, since they also need glucose to survive.

Without exception, solid tumors present high acidity in their cell tissue, characterized by the presence of hydrogen ions (H⁺). It is well known that when solid tumors reach a certain size, the penetration of oxygen in the same becomes limited. Under such conditions, oxidative phosphorylation cannot proceed normally because of insufficient oxygen, resulting in high lactic acid production, which leads to the acidification of malignant tumor tissue, allowing even its proliferation (Gatenby, R.A., Gillies, R. J. Nat. Rev. Cancer 4:891-899, 2004; Warburg, O., Review. Science 123: 309-315, 1956). See table 1 below regarding the acidity of solid tumors (a: pHi, measured by NMR-P³¹; B: pHe: Measured by glass microelectrode or potentiometry). Br. J. Cancer (1991) 64, 425-427.

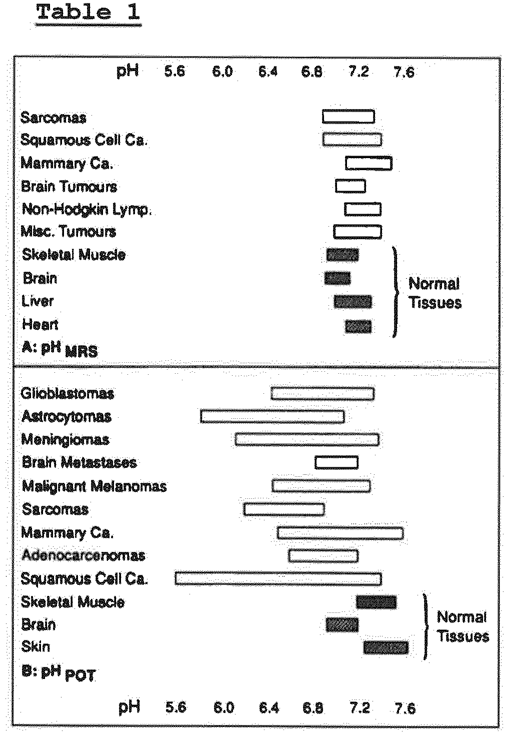

The present invention refers to a cocktail consisting of three molecules, called here "Anti-tumor cocktail", comprising an anti-tumor agent selective for the tumor acidity, an antioxidant and a P-glycoprotein inhibitor, capable of destroying selectively cells of solid tumors without affecting healthy cells of rapid replication by means of their selectivity to the acidity of the tumor cells, using a carrier of carboxyl type in the anti-tumor agent of the cocktail, which is sensitive to the acidity present in all types of malignant solid tumors. This anti-tumor compound (derived from acridine), containing a carboxylic group having a negative charge, is attracted by electrostatic forces to the interior of the tumors due to the characteristic acidity of solid tumors, which have an excess of hydrogen ions of positive charge (H⁺ ions), which attract and then neutralize the negative electrical charge of the carboxylic group of the acridine when combining with hydrogen ions H⁺ of the tumor, due to this tumor acidity, which has an excess of positive hydrogen ions, to carry the acridine drug to the DNA of the tumor cells, since only neutral molecules can very easily cross the cell membrane, leading the tumor cells to apoptosis or cellular self-destruction, while the healthy cells that multiply fast do not present acidity around them, *i.e*., they do not have the ability to attract the anti-tumor compound of negative charge, thus being spared from the destructive effects of chemotherapy.

Additionally, the present invention also uses an anti-oxidant agent, such as Coenzyme Q10, whose function is to reduce the toxicity of free radicals naturally generated by the anti-tumor agent, thus protecting healthy cells from the action of these free radicals during treatment. Moreover, the cocktail according to the invention also contains a glycoprotein P inhibitor, such as metformin, ketoconazole, quinidine, verapamil, cyclosporine, erythromycin, spironolactone, itraconazole, whose function is to cancel the resistance effect of P-glycoprotein present in large amounts in the tumor cells, responsible for pumping the chemotherapic from the inside to the outside of the tumor cells, making the concentration of these chemotherapies very low within the tumor cells which often results in the inefficacy of anti-tumoral chemotherapy.

These and other objects of the present invention will be better understood and appreciated from the detailed description of the invention and the attached claims, in which:

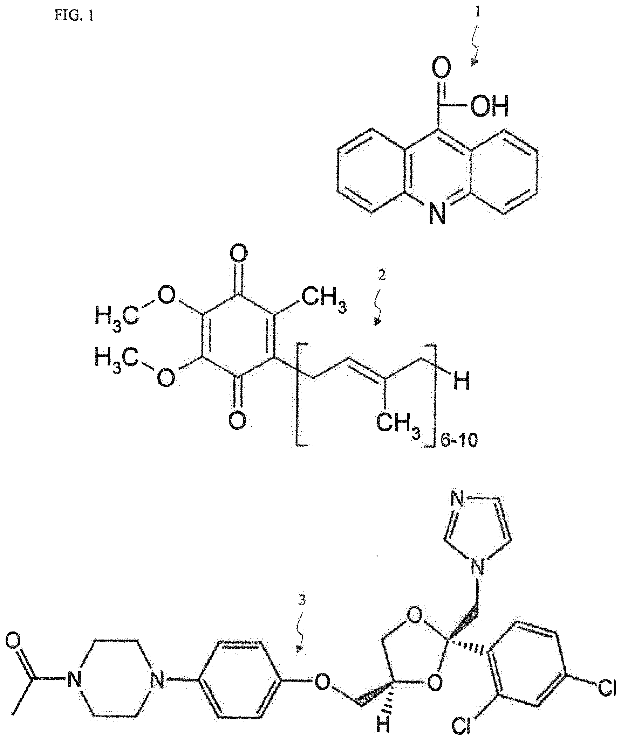

Figure 1 represents the components that the cocktail comprise, formed by the anti-tumor agent represented by 9-acridine carboxylic acid (1), the anti-oxidant agent, represented by coenzyme Q10 (2) and the P-glycoprotein inhibitor agent, represented by ketoconazole (see Formula 3 in Figure 1),or varapamil, or tariquidar, Zozuquidar, Elacridar, Laniquidar, ontogen, imidazole, antranilamide, dexverapamil or dexniguldipine. The most efficient P-glycoprotein inhibitors are tariquidar, Zozuquidar, Elacridar, or Laniquidar, considered to be the "last generation".

In accordance with the attached figure, the anti-tumor cocktail according to the present invention comprises a combination of an anti-tumor medicine (1), which may be commercial, such as 9-acridine carboxylic acid (which is illustrated by the formula (1) in the attached figure), associated with an antioxidant (2), such as coenzyme-Q10, and a P-glycoprotein inhibitor (3), such as tariquidar, and ketoconazole, in order to completely combat solid tumors without affecting the body's healthy cells. The formula (3) illustrated in the picture is ketoconazole. The said tariquidar inhibitor has the following formula:

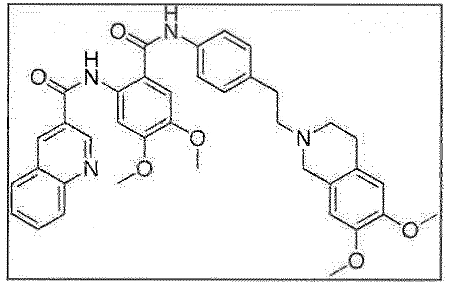

The 9-acridine carboxylic acid is a preferred anti-tumor agent because it is easily accessible on the market. However, of course, other anti-tumor agents may also be used, and physiologically acceptable salts or physiologically acceptable derivatives thereof. Therefore, the anti-tumor agent may be a compound according to the following general formula: 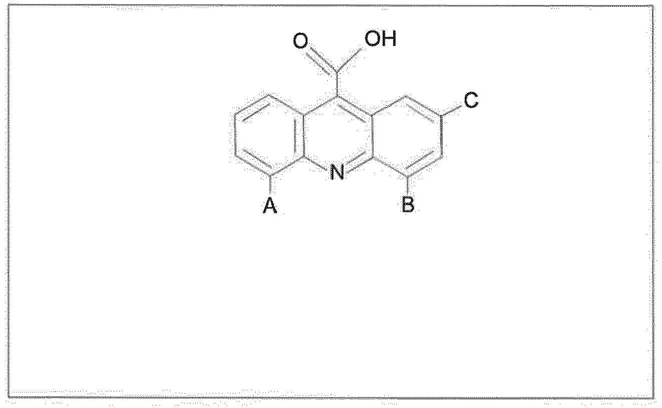
A: -OCH₃; -H; -OH; -F; -Cl
B: -H; -F; -OH; -Cl; -CH₃
C: -H; -F; -Cl; -OH; -CH₃

According to the preferred anti-tumor agent, the carboxylic group is in position 9. However, in principle it could also be in some of the positions 1, 2, 3, 4, 5, 6, 7 and 8.

The cocktail according to the present invention may consist of the three essential ingredients mentioned above, namely the anti-tumor agent selective to the tumor acidity, an antioxidant and a P-glycoprotein inhibitor. However, there may also be added various additives, depending on the circumstances, such as acceptable pharmaceutically compatible carrier materials, or diluents, as it is well known in the art. The carriers may be any inert, organic or inorganic material, suitable for enteral, percutaneous, subcutaneous or parenteral administration, such as: water, gelatin, Arabic gum, lactose, microcrystalline cellulose, starch, sodium glycinate starch, calcium hydrogen phosphate, magnesium stearate, talc, colloidal silicon dioxide, and similar. Such compositions may also contain forth pharmacologically active agents, and conventional additives such as stabilizers, wetting agents, emulsifiers, flavorizing agents, buffers, and similar. The cocktail of the present invention can also be transformed into appropriate forms, such as compositions for oral use, injections, administration in the form of nasal spray or similar, according to acceptable pharmaceutical procedures. Such pharmaceutical compositions according to the invention comprise an effective quantity of the three components, possibly in association with the various mentioned additives. The cocktails according to the invention can, e.g., be obtained in solid or liquid form for oral administration, such as tablets, pills, capsules, powders, syrups, elixirs, dispersible granules, tablets, suppositories and similar, in the form of sterile solutions, suspensions or emulsions for parental administration, sprays, e.g., nasal spray, transdermal preparations, e.g., plasters and similar.

The proportions of the contents of the three essential ingredients may vary considerably. The precise proportion is difficult to calculate because each compound depends on the body mass and the health status of the patient. Tariquidar has toxicity (LD50) equal to 300 mg/kg body (in mice) in the intravenous route. Therefore, the dosage should be below this value. For safety, we suggest a dose of 5 mg/kg body during treatment. On the other hand, Co-Q10 has toxicity (LD50) close to 20 g/kg body (orally). We suggest administering 20 mg/kg orally. Finally, the 9-acridine carboxylic acid has toxicity (LD50) equal to 240 mg/kg. We suggest using a maximum of 20 mg/kg during treatment in the intravenous route. Thus, the approximate proportion is 1:4:4 (respectively: Tariquidar; Co-Q10; and 9-acridine). Therefore, the relations between the weights of the (1) anti-tumoral agent, the (2) antioxidant and the (3) P-glycoprotein inhibitor, the ratio (1):(2):(3) of weight in a cocktail according to the present invention may be around 1:4:4. Diluents can be used as it is well known in the art. If diluents, carriers, other conventional additives such as stabilizers, wetting agents, emulsifiers, flavoring agents, buffers, and the like are used, at least 10% by weight shall consist of the three ingredients of the cocktail according to the present invention.

### EXPERIMENTS

*In vivo* assays were performed in *swiss albinos* mice in the Bioterium of the Department of Antibiotics of the Federal University of Pernambuco (Brazil). The tumors were implanted in mice following the method of Stock et al. (1955) and modified by Komiyama (1992). The mice (weighing 30g each) were divided into two groups, treated and control. The treated group received the chemotherapy by intraperitoneal injection at doses of 10 and 20 mg/kg by weight, one dose per day during the period of 6 days. On the other hand, the control group received only placebo (2% saline solution) for the same period. A total of 48 animals were used in the assays.

The purpose was to determine whether the new compound had anti-tumoral activity in the available tumors (sarcoma 180). The results were positive for the tumors, with a percentage of tumor inhibition of 48% and 90%, respectively, in the doses of 10 mg/kg and 20 mg/kg for the mice. In half of the treated group there was total remission at the dosage of 20 mg/kg.

### TOXICITY OF THE COMPONENTS OF THE ANTI-TUMORAL COCKTAIL

The tariquidar has a toxicity (LD50) equal to 300 mg/kg (in mice) when applied intravenously. Thus, its dosage should be below this value. For safety, we used a dose of 5 mg/kg during treatment.

On the other hand, Co-Q10 has a toxicity (LD50) close to 20 g/kg (orally). We used in our tests 100 mg/day orally.

Finally, the 9-acridine carboxylic acid has a toxicity (LD50) equal to 240 mg/kg, via intraperitoneal application (subcutaneously).

## Claims

1. Anti-tumor cocktail, **characterized in that** it comprises an anti-tumor agent selective for tumor acidity, an antioxidant and a P-glycoprotein inhibitor, said anti-tumoral agent comprising acridine linked to a carboxylic group.

2. Cocktail, according to claim 1, **characterized in that**, in the presence of tumor acidity, the anti-tumor agent having a negative charge will be absorbed by the tumor cells only, which have an excess of positive charge due to their acidity, saving healthy cells which have a neutral charge.

3. Cocktail, according to claim 1 or 2, **characterized in that** the said antioxidant combats the action of free radicals generated naturally by the anti-tumor agent, reducing the toxicity of the latter in relation to vital organs, such as the heart.

4. Cocktail, according to claim 1, 2 or 3, **characterized in that** said P-glycoprotein inhibitor is metformin, ketoconazole, quinidine, varapamil, ciclosporin, erythromycin, spironolactone, itraconazole, tariquidar, zozuquidar, elacridar, laniquidar, ontogen, imidazole, antranilamide, dexverapamil and/or dexniguldipine.

5. Cocktail, according to claim 4, **characterized in that** said P-glycoprotein inhibitor is tariquidar, zozuquidar, elacridar, and/or laniquidar.

6. Cocktail, according to claim 5, **characterized in that** said P-glycoprotein inhibitor is tariquidar.

7. Cocktail, according to any of the claims 1 to 6, characterized that said anti-oxidant is Coenzyme Q10.

8. Cocktail according to any one of claims 1 to 7, **characterized in that** the anti-tumor agent selective for the tumor acidity is or comprises 9-acridine carboxylic acid.

9. Cocktail according to any one of claims 1-5, **characterized in that** it is administered through any acceptable mode of administration or through agents that have similar uses, such as by oral, nasal, parenteral (intravenous, subcutaneous, intramuscular), oral, sublingual, rectal, topical, transdermal and intravesical administration.
